# FASCICULE DE BREVET EUROPEEN

(11) **EP 2 094 113 B1**
(45) Date de publication et mention de la délivrance du brevet: **14.09.2016**
(21) Numéro de dépôt: 07870287.5
(22) Date de dépôt: 15.11.2007
(51) Int. Cl.: A23L 27/40, A21D 2/14, A21D 2/36

(54) **SUBSTITUT DU SEL ET COMPOSITION PAR EXEMPLE ALIMENTAIRE LE COMPRENANT**
SALZERSATZ UND MUSTER FÜR EINE LEBENSMITTELZUSAMMENSETZUNG DAMIT
SALT SUBSTITUTE AND COMPOSITION BY FOOD EXAMPLE CONTAINING THE SAME<0}

(30) Priorité: 17.11.2006 FR 0610108
(43) Date de publication de la demande: 02.09.2009
(73) Titulaire: "R" Santé, 86310 Antigny (FR)
(72) Inventeur: RAMY, Hubert, 97400 Saint Denis De La Réunion (FR)
(74) Mandataire: Novagraaf Technologies
(86) Numéro de dépôt international: PCT/FR2007/001881
(87) Numéro de publication internationale: WO 2008/068419

(56) Documents cités:
- EP-A- 0 667 107
- WO-A-97/40705
- WO-A-98/07324
- GB-A- 1 511 200
- GB-A- 1 549 252
- JP-A- 9 238 643
- JP-A- 61 219 336
- US-A- 5 260 091
- BALDINI P ET AL: "Reduction of the quantity of NaCl used in dried meat products." VIANDES ET PRODUITS CARNES 1984 EXP. STA. FOR THE FOOD PRESERVATION IND., PARMA, ITALY, vol. 5, no. 3, 1984, pages 83-88, XP008080321
- HELLEMANN U: "Perceived taste of NaCl and acid mixtures in water and bread." INTERNATIONAL JOURNAL OF FOOD SCIENCE & TECHNOLOGY 1992 DEP. OF FOOD CHEM. & TECH., UNIV. OF HELSINKI, SF-00710, HELSINKI, FINLAND, vol. 27, no. 2, 1992, pages 201-211, XP008080290
- LAHTINEN S: "Masking of the bitter taste of salt substitutes with lactose in food emulsions." JOURNAL OF FOOD QUALITY 1986 DEP. OF FOOD CHEM. & TECH., UNIV. OF HELSINKI, SF-00710 HELSINKI, FINLAND, vol. 9, no. 4, 1986, page 199, XP008080288
- GILDBERG A ET AL: "Acceleration of autolysis during fish sauce fermentation by adding acid and reducing the salt content." JOURNAL OF THE SCIENCE OF FOOD AND AGRICULTURE 1984 INST. OF FISHERY TECH. RES. (FTFI), 9000 TROMSO, NORWAY, vol. 35, no. 12, 1984, page 1363, XP008080323

## Description

La présente invention concerne un substitut du sel, c'est-à-dire une substance capable de remplacer en totalité ou en partie le sel d'une recette en jouant le même rôle, c'est-à-dire capable de conférer au produit préparé avec ce substitut, le goût salé et les autres propriétés qualitatives et dégustatrices que lui conférerait la présence de sel.

Ainsi, l'invention concerne le domaine des compositions alimentaires, alicaments, nutraceutiques et vétérinaires dont on recherche un goût salé mais dont le sel serait absent ou en quantité bien inférieure par rapport à ce qui se pratique généralement.

L'excès de consommation de sel sous toutes ses formes est un problème de santé publique. 12 000 000 personnes dans le monde et 25 000 personnes en France meurent chaque année d'une "overdose" de sel. En effet, une consommation trop importante de sel épaissit le sang, bouche progressivement les artères, provoque de l'hypertension, éventuellement un infarctus du myocarde, un caillot dans le cerveau et donc un accident cérébral, de l'ostéoporose, de l'hypertension, des cancers, etc.

En matière d'hypertension artérielle, le sel est considéré depuis des années comme un danger majeur. L'hypertension qui affecte environ 7 millions de français est un puissant facteur de maladies cardio-vasculaires, première cause de mortalité dans l'Hexagone.

Il semble par ailleurs que l'excès de sel soit corrélé à une hypertrophie du ventricule gauche, un facteur de risque important dans le déclenchement de maladies cardio-vasculaires.

Dans le domaine de l'ostéoporose, on peut rappeler que l'os se construit grâce au calcium disponible, or tout le calcium ingéré n'est pas assimilé. Une partie du calcium part dans les urines et le sel augmente cette excrétion. Plus l'alimentation est salée, plus les fuites de calcium sont importantes.

Enfin, le sel est également incriminé dans la survenue de cancers notamment de l'estomac. Il est ainsi recommandé de ne pas faire d'excès de sel et de produits conservés en salaison, essentiellement les charcuteries.

L'excès de sel est donc plus que fortement suspecté comme étant impliqué dans l'apparition et l'aggravation de nombreuses maladies graves. Or le sel est largement utilisé dans l'industrie agroalimentaire car non seulement il n'est pas cher mais il rehausse les goûts, masque les saveurs amères et donne du relief à l'alimentation.

Ainsi, les produits transformés en comprennent une quantité excessive par rapport à ce qui est nécessaire pour l'organisme, soit selon l'OMS environ 5 g par jour.

A titre indicatif, un sandwich avec de la charcuterie et du fromage contient en moyenne 4 g de sel. Par ailleurs, on trouve beaucoup de sel dans le pain (une baguette contient environ 4 g), la charcuterie, les fromages, les pâtisseries, les céréales, les plats cuisinés, les soupes préparées, les pâtes alimentaires, les biscuits et gâteaux d'apéritifs, les cacahouètes, les anchois, les hamburgers, les pizzas, etc.

Les Français consomment environ 7,9 g de sel, voire plus tant il est difficile de mesurer exactement la quantité de sel que chacun ajoute dans son alimentation. 10 % d'entre eux en consomment plus de 12 g par jour, et certains 25 g par 24 heures.

Le document JP61219336 décrit une marinade de prunes allégée en sel dans un liquide à base de vinaigre concentré, d'une petite quantité de sel et de feuilles de la plante aromatique *Perilla frutescens var crispa.* Le vinaigre utilisé est concentré 5 fois pour atteindre une acidité de 22%.

Le document Lahtinen et al. "Masking of the bitter taste of salt substitutes with lactose in food emulsions.", JOURNAL OF FOOD QUALITY vol. 9, no. 4, 1986, page 199 Z décrit des émulsions comme la mavonnaise comprenant les substituts de sel Morton lite Salt ou Mineral Salt, dans lesquelles le goût amer de ces substituts est masqué au moyen soit de lactose, soit de vinaigre. Le vinaigre est présent à hauteur de 3 ou 6% w/w de l'émulsion.

Le document EP0667107 décrit l'ajout d'acide gluconique ou de glucono-δ-lactone à des aliments en remplacement du sel et pour promouvoir la croissance des bifidobactéries dans les aliments, et pour inhiber la croissance de bactéries délétères. Ce document indique de manière incidente qu'une solution précédemment utilisée est le remplacement du sel par du vinaigre dans les marinades de prunes, mais que cette solution de remplacement n'est pas efficace : le goût de cet aliment serait affecté de manière significative.

Il existe donc un besoin urgent de disposer d'une substance apte à remplacer en totalité ou en partie le sel de façon à limiter la quantité de sel dans ces compositions tout en préservant leur goût salé.

Ce but est atteint selon l'invention grâce à l'utilisation de vinaigre en tant que substitut de tout ou partie du sel utilisé pour la fabrication d'une composition.

L'invention est décrite le mieux par les revendications. Utilisation de vinaigre en tant que substitut de tout ou partie du sel d'une composition alimentaire, caractérisée en ce que ladite composition alimentaire est choisie dans le groupe consistant en le pain, la viennoiserie, les pâtes feuilletées, brisées et sablées, la biscuiterie, les levains, et les pâtes alimentaires, en ce que le vinaigre est dosé à environ 3 à 10% d'acidité et en ce que le vinaigre remplace au moins 60 % de la quantité de sel contenu dans la composition tout en conservant à celle-ci sa valeur gustative. Il peut s'agir d'une composition alimentaire, alicament, nutraceutique et vétérinaires, ou d'une composition intermédiaire entrant dans la constitution de ces compositions.

L'invention concerne bien entendu tous les types de vinaigres et notamment les vinaigres de vin ou de cidre, et plus généralement les vinaigres de fruits, de céréales, de malt, de riz, de maïs, de lait, etc. Ainsi, on peut citer le vinaigre dosé à environ 3 à 10% d'acidité de préférence à 5% d'acidité.

Le vinaigre est une solution aqueuse riche en acidité résultant d'une fermentation du vin ou d'un autre liquide alcoolisé. Ainsi, il comprend des particules et substances provenant de la plante et lui conférant ses propriétés diverses.

Le vinaigre selon l'invention peuvent se présenter sous forme liquide ou solide, par exemple sous forme de poudre sèche par exemple lorsqu'il s'agit d'un produit déshydraté, voire lyophilisé. Il peut s'agir ainsi d'une forme déshydratée libre ou associée à un support notamment alimentaire comme de la farine ou un autre produit comme la maltodextrine par exemple. Mais il peut aussi s'agir d'autres supports tels que cosmétique ou pharmaceutique par exemple selon le domaine d'application de la composition.

A titre d'exemple, dans une composition à base de farine, le vinaigre sous forme liquide peut être utilisé en remplacement de tout ou partie du sel à raison de 0,2 % à 5 % du poids de farine, soit de l'ordre de 2 à 50 g par kg de farine. Dans le cas de vinaigre sous forme déshydratée, celui-ci peut être utilisé à raison de 1 litre pour environ 95 g de farine, soit de l'ordre de 0,05 à 5 % du poids de farine, soit encore de 0,5 à 50 g par kilo de farine.

L'utilisation de vinaigre selon la présente invention est remarquable en ce qu'elle permet de diminuer d'au moins 60 % la quantité de sel généralement contenu dans les aliments tout en conservant leur propriété et notamment leur valeur gustative.

L'invention est remarquable en ce que le vinaigre permet également de diminuer d'au moins 33% la quantité de levure dans tous les produits de boulangerie et jusqu'à de l'ordre de 40 % voire plus du sucre dans tous les produits sucrés. L'utilisation de vinaigre à la place du sel permet aussi sur un plan nutritionnel de réduire de 30% environ l'augmentation de la glycémie induite par rapport à un pain ordinaire, et de constater une absorption plus faible et plus lente (30% environ) des sucres présents dans les produits de panification.

Le vinaigre peut être utilisé selon l'invention en remplacement de tout ou partie du sel de la composition sans modification substantielle du procédé de préparation. L'homme du métier est à même de déterminer les variantes de ces procédés de préparation susceptibles de permettre le remplacement du sel par le vinaigre sans modifier l'aspect ou les autres propriétés gustatives des compositions.

Un exemple d'une telle composition est par exemple de la farine associée à du vinaigre déshydraté.

L'invention concerne aussi la fabrication de ces compositions consistant à incorporer le vinaigre à la place de tout ou partie du sel habituellement présent.

L'utilisation de vinaigre selon l'invention permet de supprimer les aliments alcalinisant tels que les sucres qui sont abondamment utilisés dans les boulangeries, pâtisseries, biscuiteries, viandes, poissons, fruits et légumes

Un autre avantage de l'invention réside dans le fait que la présence de vinaigre dans les entrepôts de farine, aussi bien chez les artisans que chez les meuniers ou les industriels, évite l'utilisation d'insecticides, pour annihiler tous les insectes tels que charançons, mites, sylvains, etc.

En outre, L'utilisation de vinaigre selon l'invention permet de diminuer et même de supprimer le sel dans les saumures servant notamment à la conservation des charcuteries et des viandes.

D'autres avantages et caractéristiques de l'invention apparaîtront des exemples qui suivent concernant l'utilisation de vinaigre comme substitut de tout ou partie du sel dans la préparation de produits de boulangerie. Dans les exemples qui suivent, le vinaigre utilisé est le vinaigre de cidre qui est désigné dans les exemples « produit R », mais tout autre vinaigre ou équivalent de celui-ci à l'exception de ceux présentant une très forte acidité comme indiqué précédemment, peut être utilisé.

Le pain est le produit le plus représentatif des produits de boulangerie. Le pain traditionnel contient de l'ordre de 20g de sel par kilo de farine, et engendre des risques importants pour la santé. Les exemples ci-après montre que l'emploi du produit R selon l'invention permet de remplacer la majeure partie du sel, de diminuer la levure de 33% et les sucres de 40% et ainsi de prévenir les risques pour la santé.

### EXEMPLE 1 : Fabrication de pain traditionnel

Pour cette batterie d'essais le produit R a été utilisé sous forme liquide d'origine culture BIO dosé à 5% d'acidité.

### I - Recettes pour du pain traditionnel

### 1) Recette témoin du Boulanger

| | |
|---|---|
| Farine Type 65 | : 1 kg |
| Levure | : 30g |
| Eau | : 620g |
| Sel | : 20g |
| Produit R | : 0 g |
| Améliorant(Stabilisateur) | : 4g |
| - Pétrissage : | |
| 1ère Vitesse : | : 3mn |
| 2ème Vitesse | : 6m |
| - Diviser, Façonner | |
| - Pointage | : 80 mn |
| - Température | : 22° C |
| - Cuisson Four à soles | : 20 mn à 240° C |

### 2) Recette avec le produit R liquide (essai 1)

| | |
|---|---|
| Farine Type 65 | : 1 kg |
| Levure | : 20g |
| Eau | : 620g |
| Sel | : 8g |
| Produit R liquide | : 8g |
| Améliorant(Stabilisateur) | : 4g |
| - Pétrissage : | |
| 1ère Vitesse | : 3mn |
| 2ème Vitesse | : 6mn |
| - Diviser, Façonner | |
| - Pointage | : 80 mn |
| - Température | : 22° C |
| - Cuisson Four à soles | : 20 mn à 240° C |

### 3) Recette avec le produit R liquide (essai 2)

| | |
|---|---|
| Farine Type 65 | : 1 kg |
| Levure | : 20g |
| Eau | : 620g |
| Sel | : 8g |
| Produit R liquide | : 16g |
| Améliorant (Stabilisateur) | : 8g |
| - Pétrissage : | |
| 1ère Vitesse | : 3mn |
| 2ème Vitesse | : 6mn |
| - Diviser, Façonner | |
| - Pointage | : 80 mn |
| - Température | : 22° C |
| - Cuisson Four à soles | : 20 mn à 240° C |

### II - recette pour du pain traditionnel préparé la veille et pour une cuisson le lendemain

### 1) Recette témoin du Boulanger

| | |
|---|---|
| Farine Type 65 | : 1 kg |
| Levure | : 30g |
| Eau | : 620g |
| Sel | : 20g |
| Produit "R" | : 0 g |
| Améliorant(Stabilisateur) | : 4g |
| - Pétrissage : | |
| 1ère Vitesse | : 3mn |
| 2ème Vitesse | : 6mn |
| - Diviser, Façonner | |
| - Fermentation | : 18h en Chambre |
| - Température | : 22° C |
| - Cuisson Four à soles | : 20 mn à 240° C |

### 2) Recette avec le produit R liquide (essai 1)

| | |
|---|---|
| Farine Type 65 | : 1 kg |
| Levure | : 20g |
| Eau | : 620g |
| Sel | : 8g |
| Produit "R" | : 12g |
| Améliorant(Stabilisateur) | : 4g |
| - Pétrissage : | |
| 1ère Vitesse | : 3mn |
| 2ème Vitesse | : 6mn |
| - Diviser, Façonner | |
| - Fermentation | : 18h en Chambre |
| - Température | : 22° C |
| - Cuisson Four à soles | : 20 mn à 240° C |

### III - Conclusions

Pendant le processus de fabrication, l'adjonction de produit R aux différents dosages testés n'a révélé aucune nuisance dans les processus de mélange (quelque soit l'ordre de mélange des différents ingrédients) lors des opérations de pétrissage, de pointage et de cuisson.

Aspect olfactif : Il n'y a pas de différence d'odeur entre le témoin et les différentes recettes avec le produit R, les parfums sont identiques en saveur et en intensité.

Aspect Physique : Les recettes testées avec les 3 dosages de produits R ont permis d'obtenir des produits d'une texture et d'un alvéolage parfait, identique au témoin. La couleur de la croute et de la mie ne révèle aucune différence par rapport à la recette habituellement réalisée par le boulanger.

Aspect gustatif : Aucune différence de gout notable ne s'est révélée à la dégustation des 3 essais et du témoin.

L'adjonction de produit R dans la recette permet donc une diminution de quantité des levures de 33% ainsi que la suppression de 60% du sel.

### EXEMPLE 2: Fabrication de viennoiseries (Pain au chocolat, Croissant, pain aux raisins, chausson aux pommes, baguette, pâte feuilletée, pâté à la viande, maccatias, etc.)

Pour cette batterie d'essais, le produit R a été utilisé sous forme liquide d'origine culture BIO dosé à 5% d'acidité.

### 1) Recette témoin du Boulanger avec 20 heures de congélation

| | |
|---|---|
| Farine Type 65 | : 1 kg |
| Levure | : 30g |
| Sucre en poudre | : 120g |
| Eau | : 500g |
| Beurre | : 500g |
| Sel | : 20g |
| Produit R | : 0g |
| - Pétrissage | |
| 1ère Vitesse | : 3mn |
| 2ème Vitesse | : 5mn |
| - Fermentation Frigo | : 18h |
| - Température | : 4°C |
| - Diviser, Façonner | |
| - Congélation | : 20 h |
| - Température | : moins20°C |
| - Cuisson Four Ventilé | : 18 mn à 175°C |

### 2) Recette avec le produit R avec 20 heures de congélation

| | |
|---|---|
| Farine Type 65 | : 1 kg |
| Levure | : 25g |
| Sucre en Poudre | : 72g |
| Eau | : 500g |
| Beurre | : 500g |
| Sel | : 8g |
| Produit R | : 12g |
| - Pétrissage | |
| 1ère Vitesse | : 3mn |
| 2ème Vitesse | : 5mn |
| - Fermentation Frigo | : 18h |
| - Température | : 4°C |
| - Diviser, Façonner | |
| - Congélation | : 20 h |
| - Température | : moins20°C |
| - Cuisson Four Ventilé | : 18 mn à 175°C |

### 3) Recette témoin du Boulanger avec 120 heures de congélation

| | |
|---|---|
| - Farine Type 65 | : 1 kg |
| - Levure | : 30g |
| - Sucre en poudre | : 120g |
| - Eau | : 500g |
| - Beurre | : 500g |
| - Sel | : 20g |
| - Produit R | : 0g |
| - Pétrissage : | |
| 1ère Vitesse | : 3mn |
| 2ème Vitesse | : 5mn |
| - Fermentation Frigo | : 18h |
| - Température | : 4°C |
| - Diviser, Façonner | |
| - Congélation | : 120 h |
| - Température | : moins20°C |
| - Cuisson Four Ventilé | : 18 mn à 175° C |

### 4) Recette avec le produit R avec 120 heures de congélation

| | |
|---|---|
| Farine Type 65 | : 1kg |
| Levure | : 25g |
| Sucre en Poudre | : 72g |
| Eau | : 500g |
| Beurre | : 500g |
| Sel | : 8g |
| Produit "R" | : 12g |
| - Pétrissage : | |
| 1ère Vitesse | : 3mn |
| 2ème Vitesse | : 5mn |
| - Fermentation Frigo | : 18h |
| - Température | : 4°C |
| - Diviser, Façonner | |
| - Congélation | : 120h |
| - Température | : moins 20°C |
| - Cuisson Four Ventilé | : 18 mn à 175° C |

### 5) Conclusions

Pendant le processus de fabrication, l'adjonction de produit R aux différents dosages testés n'a révélé aucune nuisance dans les processus de mélange (quelque soit l'ordre de mélange des différents ingrédients) lors des opérations de pétrissage, de pointage et de cuisson.
- Aspect Olfactif : Il n'y a pas de différence d'odeur entre le témoin et les différentes recettes avec le produit R, les parfums sont identiques en saveur et en intensité.

Aspect Physique : Les pâtes des 2 tests et de la recette témoin ne présente aucune différence de texture et de couleur.

Aspect gustatif : Les Croissant et pain au chocolat des 2 tests ainsi que ceux de la recette témoins ne révèle aucune différence de goût.

L'adjonction de produit R dans la recette permet une diminution des levures de 15%, la suppression de 60% du sel ainsi qu'une diminution du sucre de 40%.

Les mêmes essais ont été réalisés en frais sans congélation, les résultats sont identiques.

### EXEMPLE 3 : Utilisation du produit R dosé à 5% d'acidité, déshydraté et fixé sur de la farine (1 litre de Produit R fixé sur 95g de farine Alimentaire)

Pour cette batterie d'essais, le Produit R a été utilisé sous forme liquide, d'origine culture BIO dosé à 5% d'acidité et déshydraté sur 95g de farine alimentaire.

### 1) Recette traditionnelle du Boulanger (Essai Témoin)

| | |
|---|---|
| Farine Type 65 | : 1kg |
| Levure | : 30g |
| Eau | : 620g |
| Sel | : 20g |
| Produit R déshydraté | : 0g |
| Améliorant (Stabilisateur) | : 4g |
| - Pétrissage : | |
| 1ère Vitesse | : 3mn |
| 2ème Vitesse | : 5mn |
| - Pointage | : 15mn |
| - Température | : 22°C |
| - Diviser, Façonner | |
| - Fermentation en Chambre | : 45mn |
| - Température | : 22°C |
| - Cuisson Four à soles | : 25mn à 240°C |

### 2) Recette avec le produit R liquide (témoin)

| | |
|---|---|
| Farine Type 65 | : 1kg |
| Levure | : 30g |
| Eau | : 620g |
| Sel | : 8g |
| Produit R Liquide | : 12g |
| Améliorant (Stabilisateur) | : 4g |
| - Pétrissage : | |
| 1ère Vitesse | : 3mn |
| 2ème Vitesse | : 5mn |
| - Pointage | : 15mn |
| - Température | : 22° C |
| - Diviser, Façonner | |
| - Fermentation en Chambre | : 45mn |
| - Température | : 22°C |
| - Cuisson Four à soles | : 25mn à 240° C |

### 3) Recette avec le produit R (essai 1)

| | |
|---|---|
| Farine Type 65 | : 1kg |
| Levure | : 20g |
| Eau | : 620g |
| Sel | : 8g |
| Produit R déshydraté | |
| et fixé sur la farine | : 2g |
| Améliorant (Stabilisateur) | : 4g |
| - Pétrissage : | |
| 1ère Vitesse | : 3mn |
| 2ème Vitesse | : 5mn |
| - Pointage | : 15mn |
| - Température | : 22°C |
| - Diviser, Façonner | |
| - Fermentation en Chambre | : 45mn |
| - Température | : 22°C |
| - Cuisson Four à soles | : 25mn à 240°C |

### 3) Recette avec le produit R(essai 2)

| | |
|---|---|
| Farine Type 65 | : 1kg |
| Levure | : 20g |
| Eau | : 620g |
| Sel | : 8g |
| Produit "R" déshydraté et fixé sur la farine | : 4g |
| Améliorant (Stabilisateur) | : 4g |
| - Pétrissage : | |
| 1ère Vitesse | : 3mn |
| 2ème Vitesse | : 5mn |
| - Pointage | : 15mn |
| - Température | : 22°C |
| - Diviser, Façonner | |
| - Fermentation en Chambre | : 45mn |
| - Température | : 22°C |
| - Cuisson Four à soles | : 25mn à 240°C |

### 4) Recette avec le produit R (essai 3)

| | |
|---|---|
| Farine Type 65 | : 1kg |
| Levure | : 20g |
| Eau | : 620g |
| Sel | : 8g |
| Produit R déshydraté et fixé sur la farine | : 6g |
| Améliorant (Stabilisateur) | : 4g |
| - Pétrissage : | |
| 1ère Vitesse | : 3mn |
| 2ème Vitesse | : 5mn |
| - Pointage | : 15mn |
| - Température | : 22°C |
| - Diviser, Façonner | |
| - Fermentation en Chambre | : 45mn |
| - Température | : 22°C |
| - Cuisson Four à soles | : 25mn à 240°C |

### 5) Recette avec le produit R (essai 4)

| | |
|---|---|
| Farine Type 65 | : 1kg |
| Levure | : 20g |
| Eau | : 620g |
| Sel | : 0g |
| Produit R déshydraté et fixé sur la farine | : 4g |
| Améliorant (Stabilisateur) | : 4g |
| - Pétrissage : | |
| 1ère Vitesse | : 3mn |
| 2ème Vitesse | : 5mn |
| - Pointage | : 15mn |
| - Température | : 22°C |
| - Diviser, Façonner | |
| - Fermentation en Chambre | : 45mn |
| - Température | : 22°C |
| - Cuisson Four à soles | : 25mn à 240°C |

### 6) Conclusions

Pendant le processus de fabrication l'adjonction de produit R aux différents dosages testés n'a révélé aucune nuisance dans les processus de mélange (quelque soit l'ordre de mélange des différents ingrédients) lors des opérations de pétrissage, de pointage et de cuisson.

Aspect Olfactif : Il n'y a pas de différence d'odeur entre le témoin et les différentes recettes "R"Santé, les parfums sont identiques en saveur et en intensité.

Aspect Physique : Les recettes testées dans les essais 1, 2 et 3 ont permis d'obtenir des produits d'une texture et d'un alvéolage parfait, identique au témoin.

La couleur de la croûte et de la mie ne révèle aucune différence par rapport à la recette habituellement réalisée par le boulanger.

Aspect gustatif : Aucune différence de goût notable ne s'est révélée à la dégustation des 5 essais et du témoin. Toutefois le meilleur équilibre est atteint sur le dosage à 4g de produits R déshydraté (essai 2) et sur l'essai 5 ou nous avions totalement supprimé le sel

Que ce soit sous forme liquide ou déshydraté l'adjonction de produit R aux différentes recettes conserve les mêmes avantages, à savoir diminution du sel d'au moins 60% et baisse de 33% de la levure

D'autres essais ont été réalisés à partir de concentrations différentes (1 litre de produit R fixé sur 450g de farine et 1 litre sur 750g de farine), de taux d'acidité compris entre 4 et 10 %, ces essais ont donné des résultats identiques proportionnels aux quantités et aux pourcentages.

### EXEMPLE 4 : Esais avec l'acide acétique concentrée à 85%

(exemple de référence)

### 25 1) Recette du Boulanger avec le produit R

| | |
|---|---|
| Farine Type 65 | : 1 kg |
| Levure | : 30g |
| Eau | : 620g |
| Sel | : 20g |
| Produit R | : 0g |
| Améliorant(Stabilisateur) | : 4g |
| - Pétrissage : | |
| 1ère Vitesse | : 3mn |
| 2ème Vitesse | : 6mn |
| - Diviser, Façonner | |
| - Pointage | : 45mn |
| - Température | : 22°C |
| - Fermentation en Chambre | : 15mn |
| - Température | : 22°C |
| - Cuisson Four à soles | : 20mn à 240°C |

### 2) Essai 1 avec l'acide acétique

| | |
|---|---|
| Farine Type 65 | : 1kg |
| Levure | : 20g |
| Eau | : 620g |
| Sel | : 8g |
| Acide Acétique 85% | : 2g |
| Améliorant(Stabilisateur) | : 4g |
| - Pétrissage : | |
| 1ère Vitesse | : 3mn |
| 2ème Vitesse | : 6mn |
| - Diviser, Façonner | |
| - Pointage | : 45mn |
| - Température | : 22°C |
| - Fermentation en Chambre | : 15mn |
| - Température | : 22°C |
| - Cuisson Four à soles | : 20 mn à 240°C |

### 3) Essai 2 avec l'acide acétique

| | |
|---|---|
| Farine Type 65 | : 1kg |
| Levure | : 20g |
| Eau | : 620g |
| Sel | : 8g |
| Acide Acétique 85% | : 4g |
| Améliorant(Stabilisateur) | : 4g |
| - Pétrissage : | |
| 1ère Vitesse | : 3mn |
| 2ème Vitesse | : 6mn |
| - Diviser, Façonner | |
| - Pointage | : 80 mn |
| - Température | : 22°C |
| - Fermentation en Chambre | : 15mn |
| - Température | : 22°C |
| - Cuisson Four à soles | : 20mn à 240°C |

### 4) Essai 3 avec l'acide acétique

| | |
|---|---|
| Farine Type 65 | : 1kg |
| Levure | : 20g |
| Eau | : 620g |
| Sel | : 8g |
| Acide Acétique 85% | : 8g |
| Améliorant(Stabilisateur) | : 4g |
| - Pétrissage : | |
| 1ère Vitesse | : 3mn |
| 2ème Vitesse | : 6mn |
| - Diviser, Façonner | |
| - Pointage | : 80 mn |
| - Température | : 22° C |
| - Fermentation en Chambre | : 15mn |
| - Température | : 22°C |
| - Cuisson Four à soles | : 20 mn à 240°C |

### 5) Essai 4 avec l'acide acétique

| | |
|---|---|
| Farine Type 65 | : 1 kg |
| Levure | : 20g |
| Eau | : 620g |
| Sel | : 8g |
| Acide Acétique 85% | : 13g |
| Améliorant(Stabilisateur) | : 4g |
| - Pétrissage : | |
| 1ère Vitesse | : 3mn |
| 2ème Vitesse | : 6mn |
| - Pointage | : 80mn |
| - Température | : 22°C |
| - Fermentation en Chambre | : 15mn |
| - Température | : 22°C |
| - Cuisson Four à soles | : 20mn à 240°C |

### 6) Conclusions

Pendant le processus de fabrication l'adjonction d'acide acétique concentré à 85% au delà de 2g provoque la destruction des levures à 70% et ne permet pas une pousse correcte de la pâte.

Aspect Olfactif : Dosé a 2 g, l'acide acétique ne génère pas de différence d'odeur en comparaison du témoin. A partir de 4g d'acide acétique une forte odeur se dégage pendant les manipulations ainsi que pendant la cuisson pouvant générer des problèmes d'irritations.

Aspect Physique : Pour le test 1, soit 2g d'acide acétique, on ne constate pas de différence notoire de texture et d'alvéolage. Pour l'ensemble des autres dosages, l'acide acétique ayant provoqué la destruction des levures, la pousse de la pâte a totalement été annihilée et produit donc un pain totalement impropre à la consommation

Aspect gustatif : Sur le test 1, soit 2g d'acide acétique, le gout du pain est plus fade que le témoin. Pour l'ensemble des autres tests l'augmentation de la dose d'acide acétique génère un gout amer d'une intensité proportionnel à la dose d'acide

Il est donc possible de considérer que la substitution du produit R par de l'acide acétique ne permet pas de garder les qualités physique, olfactive et gustative de la recette témoin et produit un pain impropre a la consommation.

## Revendications

1. Utilisation de vinaigre en tant que substitut de tout ou partie du sel d'une composition alimentaire, **caractérisée en ce que** ladite composition alimentaire est choisie dans le groupe consistant en le pain, la viennoiserie, les pâtes feuilletées, brisées et sablées, la biscuiterie, les levains, et les pâtes alimentaires, **en ce que** le vinaigre est dosé à environ 3 à 10% d'acidité et **en ce que** le vinaigre remplace au moins 60 % de la quantité de sel contenu dans la composition tout en conservant à celle-ci sa valeur gustative.

2. Utilisation selon la revendication 1, **caractérisée en ce que** le vinaigre est choisi parmi le vinaigre de vin ou de cidre ou plus généralement de fruits, de céréales, de malt, de riz, de maïs, de lait, etc.

3. Utilisation selon l'une quelconque des revendications 1 ou 2, **caractérisée en ce que** le vinaigre se présente sous forme liquide ou solide, par exemple sous forme de poudre sèche par exemple lorsqu'il s'agit d'un produit déshydraté.

4. Utilisation selon l'une quelconque des revendications précédentes, **caractérisée en ce que** le vinaigre se présente sous forme déshydratée libre ou fixée sur un support alimentaire comme de la farine ou la maltodextrine par exemple.

5. Utilisation selon l'une quelconque des revendications précédentes, **caractérisée en ce que** dans une composition à base de farine, le vinaigre sous forme liquide est utilisé en remplacement de tout ou partie du sel à raison de 0,2 % à 5 % du poids de farine, soit de l'ordre de 2 à 50 g par kg de farine, et dans le cas de vinaigre ou équivalent de celui-ci sous forme déshydratée, celui-ci est utilisé à raison de 1 litre pour environ 95 g de farine, soit de l'ordre de 0,05 à 5 % du poids de farine, soit encore de 0,5 à 50 g par kilo de farine.

## Patentansprüche

1. Verwendung von Essig als Ersatz für alles oder einen Teil des Salzes in einer Nahrungsmittelzusammensetzung, **dadurch gekennzeichnet, dass** die Nahrungsmittelzusammensetzung aus der Gruppe ausgewählt ist, die aus Brot, Feingebäck, Blätter-, Mürbe- und Sandteig, Keksen, Fermenten und Teigwaren besteht und dass der Essig mit einem Säuregehalt von 3 bis 10 % dosiert ist und dass der Essig mindestens 60 % der in der Zusammensetzung enthaltenen Salzmenge bei Bewahrung des Geschmackwerts derselben ersetzt.

2. Verwendung nach Anspruch 1, **dadurch gekennzeichnet, daß** der Essig unter Wein- oder Apfelessig ausgewählt ist oder allgemeiner aus Obst-, Getreide-, Malz-, Reis-, Mais-, Milchessig, etc.

3. Verwendung nach einem der Ansprüche 1 oder 2, **dadurch gekennzeichnet, daß** der Essig in flüssiger oder fester Form vorliegt, zum Beispiel in Form eines Trockenpulvers, wenn es sich um ein Trockenprodukt handelt.

4. Verwendung nach einem der vorhergehenden Ansprüche, **dadurch gekennzeichnet, daß** der Essig in freier getrockneter oder in an einen Lebensmittelträger, wie zum Beispiel Mehl oder Maltodextrin, gebundener Form vorliegt.

5. Verwendung nach einem der vorhergehenden Ansprüche, **dadurch gekennzeichnet, daß** in einer Zusammensetzung auf Mehlbasis der Essig in flüssiger Form als Ersatz eines Teils oder allen Salzes in einer Menge von 0,2 % bis 5 % des Mehlgewichts, d.h. in einer Größenordnung von 2 bis 50 g pro kg Mehl verwendet wird und, in dem Fall, dass der Essig oder ein Äquivalent von diesem in getrockneter Form vorliegt, dieser in einer Menge von 1 l für ungefähr 95 g Mehl, d.h. in einer Größenordnung von 0,05 bis 5 % des Mehlgewichts, d.h. auch 0,5 bis 50 g pro Kilo Mehl, verwendet wird.

## Claims

1. Use of vinegar as a substitute of all or part of the salt of a food composition, **characterized in that** said composition is selected in the group consisting of bread, Viennese pastry, puff pastries, short pastries and shortbreads, biscuits, sourdoughs, and pastas, **in that** the vinegar has an acidity of about 3 to 10% and **in that** the vinegar replaces at least 60% of the salt quantity contained in the composition while preserving the food composition gustative values.

2. Use according to claim 1, **characterized in that** the vinegar is selected among wine vinegar, or cider vinegar, or more generally fruit vinegar, cereal vinegar, malt vinegar, rice vinegar, corn vinegar, milk vinegar, etc.

3. Use according to anyone of claims 1 or 2, **characterized in that** the vinegar is in a liquid or a solid form, for example a dry powder for example when it is a dehydrated form.

4. Use according to anyone of the preceding claims, **characterized in that** the vinegar is in a free dehydrated form or a dehydrated form fixed on a food support as flour or maltodextrine for example.

5. Use according to anyone of the preceding claims, **characterized in that** in a flour-based composition, the vinegar in a liquid form is used for replacing all or part of the salt at a rate of 0.2% to 5% of flour weight, that is in an order of 2 to 50 kg per flour kg, and in the case of vinegar or an equivalent thereof, in a dehydrated form, it is used at a rate of 1 liter per about 95 g of flour, that is in the order of 0.5 to 50 g per flour kilo.
